# EUROPÄISCHE PATENTANMELDUNG

(11) **EP 0 672 888 A2**
(43) Veröffentlichungstag der Anmeldung: **20.09.1995**
(21) Anmeldenummer: 95102820.8
(22) Anmeldetag: 28.02.1995
(51) Int. Cl.: G01B 11/08, B07C 5/10, B08B 9/08

(54) **Verfahren und Vorrichtung zur Überprüfung von Gebinden**

(30) Priorität: 16.03.1994 DE 4408948
(71) Anmelder: GEA Till GmbH & Co., D-65830 Kriftel (DE)
(72) Erfinder: Till, Volker, Dipl. Ing., D-65719 Hofheim am Taunus (DE); Woite, Rainer, Dipl. Ing., D-65824 Schwalbach am Taunus (DE)
(74) Vertreter: KEIL & SCHAAFHAUSEN Patentanwälte

(57) **Zusammenfassung**

Die Anmeldung betrifft ein Verfahren und eine Vorrichtung zur Überprüfung von Gebinden, insbesondere von Fässern wie Kegs. Zur Feststellung von Deformationen des Fasses ist vorgesehen, daß mit wenigstens einer Kamera ein Bild eines zu überprüfenden Abschnitts des Fasses, insbesondere einer Bodenfläche des Fasses mit einem Faßkragen, aufgenommen wird, daß die Bilddaten erfaßt und an eine Datenverarbeitungseinrichtung weitergeleitet werden, und daß die Daten in der Datenverarbeitungseinrichtung ausgewertet werden.

## Beschreibung

Die Erfindung betrifft ein Verfahren zur Überprüfung von Gebinden, insbesondere von Fässern wie Kegs, sowie eine Vorrichtung zur Durchführung eines solchen Verfahrens.

In der Getränkeindustrie werden wie in vielen anderen Industriezweigen Gebinde eingesetzt, die üblicherweise als Fässer, in der Getränkeindustrie, insbesondere wenn die Ventile eingeschraubt sind, als Kegs bezeichnet werden. Die Fässer bestehen üblicherweise aus rostfreiem Stahl oder aus Kombinationen von rostfreiem Stahl mit anderen Werkstoffen, wie z.B. Polyurethan oder Gummi, insbesondere im Bereich der Faßkragen oder -enden. Die Fässer sind Mehrweggebinde, die nach Entleerung beim Kunden zur Reinigung und erneuten. Befüllung in das Abfüllwerk zurückgebracht werden. Aufgrund ihres robusten Aufbaus können die Fässer in einer Vielzahl von Umläufen zwischen dem Abfüllwerk und den Benutzern verwendet werden. Mit zunehmendem Alter der Gebinde und Anzahl der Umläufe treten jedoch immer häufiger deformierte und beschädigte Fässer auf, die Störungen in den Reinigungs- und Abfüllanlagen verursachen.

Aufgabe der Erfindung ist es daher, ein Verfahren und eine Vorrichtung vorzuschlagen, die es ermöglichen, derartige Beschädigungen der Fässer festzustellen und beschädigte Gebinde vor Beginn des Reinigungs- und Füllvorgangs aus der Produktionslinie der Abfüllanlage auszuschleusen.

Diese Aufgabe wird mit der Erfindung im wesentlichen dadurch gelöst, daß mit wenigstens einer Kamera ein Bild eines zu überprüfenden Abschnitts des Fasses, insbesondere einer Bodenfläche des Fasses mit einem Faßkragen, aufgenommen wird, daß die Bilddaten erfaßt und an eine Datenverarbeitungseinrichtung weitergeleitet werden, und daß die Daten in der Datenverarbeitungseinrichtung ausgewertet werden. Die Nutzung eines Bildverarbeitungssystems zur Überprüfung der Fässer erlaubt eine weitgehend automatische Kontrolle. Wird bei der Auswertung in der Datenverarbeitungseinrichtung festgestellt, daß die Bilddaten des untersuchten Fasses außerhalb festgelegter Toleranzbereiche liegen, kann durch entsprechende Steuerung der Abfüllanlage das deformierte Faß aus der Produktionslinie ausgeschleust und einem Reparaturbereich zugeführt werden, ohne daß es zu Störungen der Abfüllanlage kommt.

Da die häufigste Beschädigung der Fässer in Form von Deformierungen des Faßkragens, bspw. beim Absetzen des Fasses, erfolgt, ist gemäß einer bevorzugten Ausgestaltung der Erfindung vorgesehen, daß an mehreren, vorzugsweise gleichmäßig über den Umfang des Fasses verteilten Stellen des von der Kamera aufgenommenen Bildes der Außen- bzw. Innendurchmesser des Faßkragens ermittelt wird, so daß radiale Verformungen festgestellt werden können. Durch die Durchmesserermittlung jedes untersuchten Fasses kann die Analyse der Deformierung des Faßkragens unabhängig von dem Faßtyp automatisch erfolgen. Damit ist eine Verarbeitung aller Faßdurchmesser ohne Kalibrierung möglich.

Eine ausreichend genaue Aussage über die Form des Faßkragens läßt sich erfindungsgemäß dadurch ermitteln, daß der Außen- und/oder Innendurchmesser des Faßkragens an jeweils 8 Stellen ermittelt wird.

In Weiterbildung des Erfindungsgedankens ist vorgesehen, daß aus den ermittelten Daten des Außen- bzw. Innendurchmessers des Faßkragens ein Mittelwert, der sogenannte Außen- bzw. Innenbestkreis, gebildet wird. Die ermittelten Bestkreise können als Ausgangspunkt für die Analyse der Deformierungen des Faßkragens verwendet werden.

Bei der erfindungsgemäßen Lösung werden die Abweichungen des ermittelten maximalen und minimalen Außendurchmessers von dem Außenbestkreis des Faßkragens festgestellt. Auch hierbei ist die Analyse für verschiedenste Faßtypen möglich, da jeweils der für das individuelle Faß gebildete Bestkreis herangezogen wird.

Auf gleiche Weise werden erfindungsgemäß die ermittelten Innendurchmesser des Faßkragens mit dem Innenbestkreis verglichen.

Da es auch vorkommen kann, daß Fässer zurückgeliefert werden, die nicht in der Abfüllanlage verarbeitbar sind oder von der Brauerei nicht vertrieben werden, ist bei einer Ausgestaltung der Erfindung vorgesehen, daß der ermittelte Außen- bzw. Innenbestkreis des Faßkragens mit in einer Datenbank gespeicherten Werten üblicher Fässer verglichen wird. Damit ist es möglich, Fremdfässer automatisch aus der Abfüllanlage auszuschleusen.

Bei einer weiteren bevorzugten Ausführungsform der Erfindung wird in dem von der Kamera aufgenommenen Bild die Anzahl der Pixel ermittelt, die außerhalb des Außenbestkreises und innerhalb des Innenbestkreises liegen sowie die Anzahl der zwischen Außen- und Innenbestkreis liegenden Pixel. Durch Analyse der ermittelten Pixelzahlen läßt sich eine eventuelle Deformierung des Faßkragens ermitteln.

Um auch die Fälle zu erfassen, bei denen eine einzelne Stelle des Faßkragens axial eingedrückt ist, wird erfindungsgemäß vorgeschlagen, das Faß auszusondern, wenn in einem Umfangsbereich des Faßkragens keine Pixel ermittelt werden.

Die axiale Deformation des Faßkragens kann aber auch in einem Schiefdrücken des Faßkragens bestehen. Erfindungsgemäß ist daher vorgesehen, daß der Abstand verschiedener Umfangsbereiche des Faßkragens zu einer Referenzebene ermittelt wird, die vorzugsweise horizontal liegt. Dadurch läßt sich eine eventuelle Schiefstellung des Faßkragens ermitteln.

Zweckmäßigerweise erfolgt die Abstandsmessung mittels eines Lasermeßgerätes oder mit Ultraschall. Es kann jedoch auch vorgesehen sein, daß die Abstandsmessung mit zwei Kameras durchgeführt wird und der Abstand durch Auswertung der Lichtreflexe, bspw. durch Stereometrie, ermittelt wird.

Es kommt auch vor, daß die von der Kamera überprüfte Seite des Fasses keine Deformationen aufweist, daß aber eine Deformation der gegenüberliegenden Seite des Fasses eine Schiefstellung des Fasses bewirkt. Bei einer Auswertung des von der nicht-deformierten Seite aufgenommenen Bildes würde sich in einem solchen Fall statt der eigentlich vorliegenden Kreisform des Faßkragens eine Ellipse ergeben. Auch die Abstandsmessung würde eine Deformation des Faßkragens anzeigen, obwohl auf dieser Faßseite tatsächlich keine Deformation vorliegt. Erfindungsgemäß wird daher vorgeschlagen, daß auf der Bodenfläche des Fasses Referenzpunkte aufgenommen werden und daß der Abstand der Referenzpunkte von der Referenzebene ermittelt wird.

Vorzugsweise werden dabei vier um jeweils 90° zueinander versetzte Referenzpunkte auf der Faßbodenfläche aufgenommen.

Da die Faßbodenfläche durch den Faßkragen geschützt ist, treten an ihr üblicherweise keine Deformationen auf, so daß sie als Basis zur Berechnung einer eventuellen Schiefstellung des Fasses genutzt werden kann. Bei der erfindungsgemäßen Losung ist vorgesehen, daß der Abstand der einzelnen Referenzpunkte auf der Faßbodenfläche von der Referenzebene zueinander in Beziehung gesetzt wird, daß die Relativlage des Faßbodens zur Horizontalen ermittelt wird, und daß bei einer ermittelten Schiefstellung des Fasses das von der Kamera aufgenommene Bild des Faßkragens um die Faßneigung korrigiert wird. Nach einer derartigen Korrektur kann überprüft werden, ob der untersuchte Faßkragen tatsächlich deformiert ist oder eine eventuelle Fehlermeldung lediglich auf eine Schiefstellung des Fasses zurückzuführen ist.

In Weiterbildung der Erfindung ist vorgesehen, daß von der Ober- und/oder der Unterseite des Fasses ein Bild aufgenommen und ausgewertet wird, und daß das Faß ausgesondert wird, wenn die ermittelten Daten der Ober- und/oder Unterseite des Fasses außerhalb bestimmter Toleranzbereiche liegen.

Die Fässer werden durch ein Faßventil gereinigt und befüllt, das als Fitting bezeichnet wird und insbesondere bei Kegs in einer Muffe eingeschraubt wird. Wird die Muffe beim Transport beschädigt, kann dies zu einer Schrägstellung des Ventils im Faß führen, so daß keine ausreichende Reinigung, Befüllung oder Entleerung möglich ist. Um bei der Überprüfung des Fasses auch derartige Deformationen erfassen zu können, wird bei einer bevorzugten Ausführungsform der Erfindung vorgeschlagen, daß der Abstand der Oberfläche eines Fittings von der Faßbodenfläche an wenigstens zwei einander vorzugsweise gegenüberliegende Referenzpunkten der Fittingoberfläche ermittelt wird, und daß das Faß ausgesondert wird, wenn die Abstände der wenigstens zwei Referenzpunkte auf der Fittingoberfläche von der Faßbodenfläche um mehr als einen festgelegten Toleranzwert voneinander abweichen. Wie oben beschrieben wird die Faßbodenfläche üblicherweise nicht deformiert, so daß bei einem unterschiedlichen Abstand der Referenzpunkte der Fittingoberfläche zu der Faßbodenfläche von einer Schiefstellung des Fittings ausgegangen werden kann.

Eine erfindungsgemäße Vorrichtung zur Durchführung des oben beschriebenen Verfahrens weist bspw. wenigstens eine Kamera zur Aufnahme von Bildern von zu überprüfenden Abschnitten der Fässer, insbesondere der Bodenfläche des Fasses mit dem Faßkragen, und eine Datenverarbeitungseinrichtung auf, in der die Bilddaten ausgewertet werden.

Die Kamera kann dabei erfindungsgemäß eine CCD-Zeilenkamera oder eine Videokamera sein.

Bei einer bevorzugten Ausgestaltung der Erfindung weist die Datenverarbeitungseinrichtung einen Speicher zur Speicherung der ermittelten Daten und einen Speicher für die Daten bekannter Faßtypen auf.

In Weiterbildung der Erfindung ist eine Meßeinrichtung für die Ermittlung des Abstandes einzelner Abschnitte des Fasses von einer Referenzebene vorgesehen, mit der eventuelle Schiefstellungen des Faßkragens ermittelbar sind.

Die Meßeinrichtung ist zweckmäßigerweise ein optisches Lasermeßgerät oder ein Ultraschallgerät.

Bei einer besonders zweckmäßigen Ausführungsform der Erfindung ist vor und nach einer Wendemaschine für das Faß jeweils eine Kamera angeordnet, die Bilder von der Ober- bzw. der Unterseite des Fasses aufnimmt. Da eine solche Wendemaschine üblicherweise am Anfang einer Abfüllanlage angeordnet ist, kann unter Ausnutzung der bestehenden Strukturen eine frühzeitige Fehlererkennung ermöglicht werden.

In Weiterbildung dieses Erfindungsgedankens ist ein Zwischenspeicher für die Daten des von der vor der Wendemaschine angeordneten Kamera aufgenommenen Bildes vorgesehen, so daß die Bilddaten beider Kameras gemeinsam ausgewertet werden können.

Weiterbildungen, Vorteile und Anwendungsmöglichkeiten der Erfindung ergeben sich aus der nachfolgenden Beschreibung von Ausführungsbeispielen und der Zeichnung. Dabei bilden alle beschriebenen und/oder dargestellten Merkmale für sich oder in beliebiger Kombination den Gegenstand der Erfindung, unabhängig von ihrer Zusammenfassung in den Ansprüchen oder deren Rückbeziehung.

Es zeigen:
- Fig. 1: eine schematische Darstellung eines zu untersuchenden Fasses mit einer darüber angeordneten Kamera,
- Fig. 2: eine schematische Darstellung eines aufgenommenen Bildes mit eingezeichneten Bestkreisen,
- Fig. 3: eine ähnliche Darstellung wie in Fig. 2 mit angedeuteten Pixeln,
- Fig. 4: ein Faß mit einer axialen Eindellung an einer Stelle,
- Fig. 5: das sich bei einem in Fig. 4 dargestellten Faß ergebende Pixelbild,
- Fig. 6: eine schematische Darstellung der Abstandsmessung des Faßkragens von einer Referenzebene,
- Fig. 7: eine schematische Seitenansicht der Meßeinrichtung,
- Fig. 8: ein schiefstehendes Faß,
- Fig. 9: ein Faß mit schiefsitzendem Fitting, und
- Fig. 10: eine erfindungsgemäße Vorrichtung mit einer Wendemaschine.

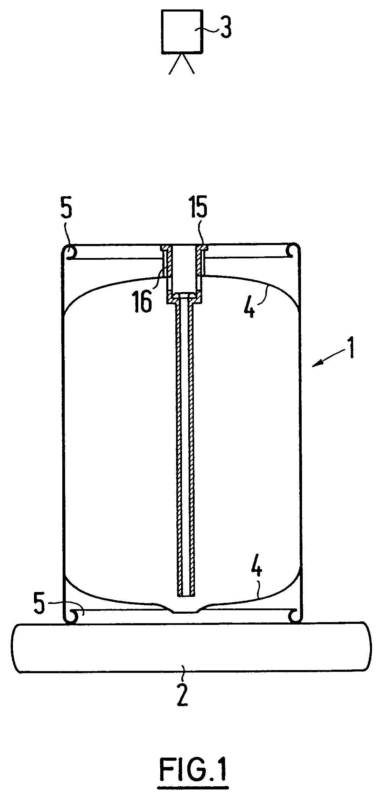

Das in Fig. 1 dargestellte Faß 1 ist ein Mehrweggebinde, das nach Entleeren beim Kunden zum Reinigen und erneuten Befüllen in die Abfüllanlage zurückgebracht wurde. Um zu vermeiden, daß Deformierungen an dem Faß 1 den Reinigungs- und/oder Füllvorgang stören und zu Unterbrechungen des Produktionsprozesses führen, wird mit dem erfindungsgemäßen Verfahren überprüft, ob das Faß 1 deformiert ist. Wird eine Deformation festgestellt, so wird das Faß 1 aus dem Produktionsfluß ausgeschleust und einem Reparaturbereich zugeführt.

Mit einer vorzugsweise über einem Transportband 2 für das Faß 1 angeordneten Kamera 3 wird ein Bild von einer Bodenfläche 4 und einem dazugehörigen Faßkragen 5 des Fasses 1 aufgenommen. Die Bilddaten werden an eine nicht dargestellte Datenverarbeitungseinrichtung weitergeleitet und dort ausgewertet.

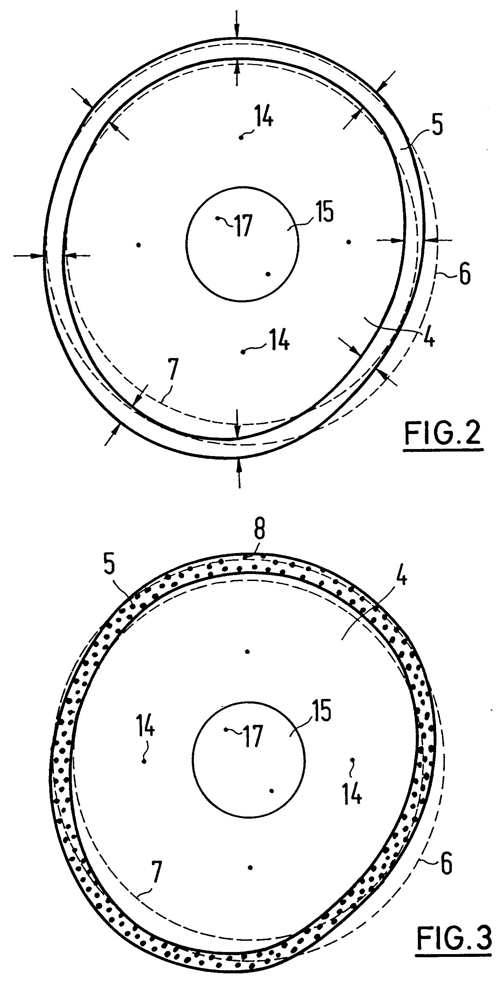

In Fig. 2 ist das von der Kamera 3 aufgenommene Bild schematisch dargestellt. Der Faßkragen 5 ist über seinen Umfang unregelmäßig deformiert und weicht von der ursprünglichen Kreisform eines neuen Fasses ab. In dem aufgenommenen Bild werden an acht, in Fig. 2 durch Pfeile angedeuteten Stellen die Außen- und Innendurchmesser des Faßkragens 5 ermittelt. Aus den erhaltenen Werten wird in der Datenverarbeitungseinrichtung ein Mittelwert gebildet, der in einen Außenbestkreis 6 und einen Innenbestkreis 7 umgesetzt wird. Die Bestkreise 6, 7 sind in der Zeichnung gestrichelt dargestellt. Bei der Darstellung in Fig. 2 steht der Faßkragen 5 in Teilbereichen über den Außenbestkreis 6 bzw. den Innenbestkreis 7 vor.

Nun werden für jeden gemessenen Außen- und Innendurchmesser des Faßkragens 5 die maximalen und minimalen Abweichungen von dem Außenbestkreis 6 und dem Innenbestkreis 7 ermittelt. Ergibt sich, daß die Abweichungen außerhalb eines festgelegten Toleranzbereiches liegen, so ist das Faß 1 so stark deformiert, daß eine ordnungsgemäße Reinigung oder Füllung nicht mehr gewährleistet ist. Das deformierte Faß 1 wird ausgesondert und einem Reparaturbereich zugeführt.

In der Datenverarbeitungseinrichtung ist ein Speicher vorgesehen, der die Außen- und Innendurchmesser der Faßkragen des bekannten Faßbestandes enthält. Diese Daten werden nun mit dem für das Faß 1 ermittelten Außenbestkreis 6 und Innenbestkreis 7 verglichen. Stellt sich heraus, daß die Daten des Fasses 1 um mehr als einen festgelegten Toleranzbereich von den im Speicher der Datenverarbeitungseinrichtung enthaltenen Daten abweichen, so handelt es sich bei dem Faß 1 um ein Fremdfaß, daß für die Abfüllanlage nicht geeignet ist oder von der Brauerei üblicherweise nicht vertrieben wird. In einem solchen Fall wird das Faß 1 ausgesondert.

Bei einem etwas anderem Verfahren zur Auswertung des von der Kamera 3 aufgenommenen Bildes werden, wie in Fig. 3 dargestellt, Pixel 8 der Abbildung des Faßkragens 5 ermittelt.

Dabei wird sowohl die Anzahl der Pixel 8 ermittelt, die außerhalb des Außenbestkreises 6 und innerhalb des Innenbestkreises 7 liegen als auch die Anzahl der zwischen dem Außenbestkreis 6 und dem Innenbestkreis 7 liegenden Pixel. Sollte das Verhältnis der innerhalb und außerhalb der Bestkreise 6, 7 liegenden Pixel 8 einen festgelegten Toleranzbereich verlassen, so wird das Faß 1 ausgesondert und dem Reparaturbereich zugeführt.

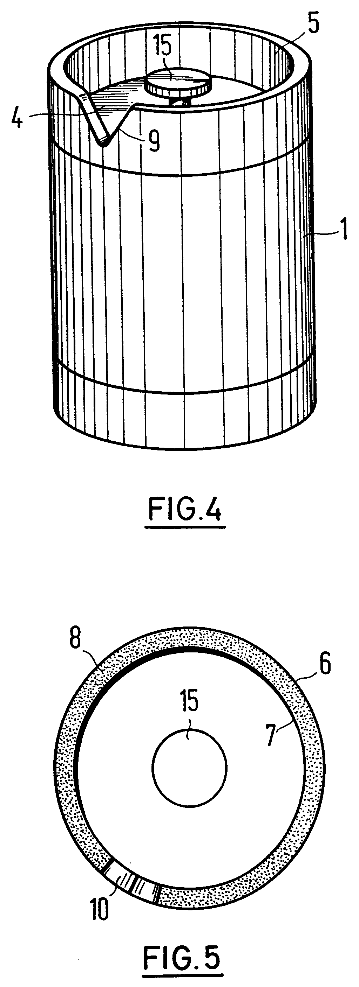

Durch die oben beschriebenen Verfahren werden im wesentlichen Faßkragendeformationen in radialer Richtung erfaßt. Es ist aber auch möglich, daß der Faßkragen 5 in Axialrichtung deformiert ist. Ein derart deformiertes Faß 1 ist in Fig. 4 schematisch dargestellt. Eine axiale Eindellung 9 ist durch Analyse der Außen- und Innendurchmesser des Faßkragens 5 nicht feststellbar, da sich diese bei einer rein axialen Verformung nicht ändern. Bei der Ermittlung der Pixel 8 in der Ebene des Faßkragens 5 ergibt sich jedoch im Bereich der Eindellung 9 eine Lücke 10, wie in Fig. 5 dargestellt. Wird bei der Analyse des von der Kamera 3 aufgenommenen Bildes eine derartige Lücke 10 festgestellt, so wird das Faß 1 ausgesondert.

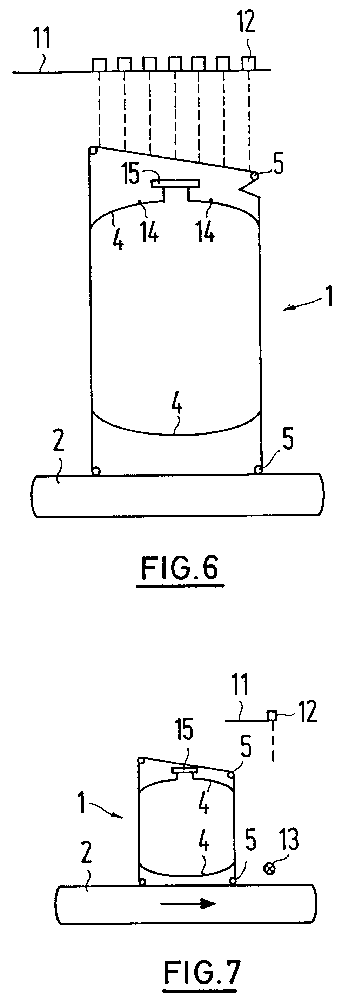

Ist, wie in Fig. 6 dargestellt, der gesamte Faßkragen 5 schief eingedrückt, so läßt sich die Deformation nur durch eine Abstandsmessung des Faßkragens 5 von einer horizontalen Referenzebene 11 feststellen. Die Abstandsmessung erfolgt über Meßeinrichtungen 12, die über dem Faß 1 angeordnet sind und den Abstand zwischen dem Faßkragen 5 und der Referenzebene 11 erfassen. Die Meßeinrichtungen 12 können optische Lasermeßgeräte oder Ultraschallgeräte sein. Auch ist es möglich, zwei Kameras vorzusehen, mit denen die Lichtreflexe, ggf. mittels Stereometrie, ausgewertet werden. Da die Meßeinrichtungen 12 im wesentlichen punktförmig arbeiten, ist eine Vielzahl von Meßeinrichtungen 12 quer zu dem Transportband 2 des Fasses 1 angeordnet. Über eine Lichtschranke 13 wird der Beginn der Messung ausgelöst. Die Meßzellen 12 speichern die gemessenen Werte während des Durchlaufs des Fasses 1 und übermitteln die Werte an die Datenverarbeitungseinrichtung. Wird eine Schiefstellung des Faßkragens 5 ermittelt, so wird das Faß 1 ausge- sondert.

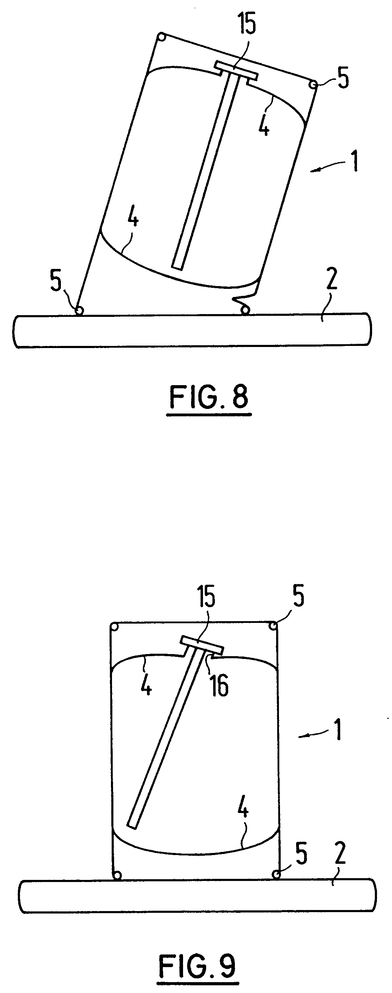

Ist die der Kamera 3 zugeordnete Seite des Fasses 1 nicht deformiert, dafür aber die gegenüberliegende Seite des Fasses, so steht ggf. das komplette Faß 1 schief. Ein derart deformiertes Faß 1 ist in Fig. 8 dargestellt. In einem solchen Fall wird sowohl bei der Abstandsmessung mit den Meßeinrichtungen 12 als auch bei der Analyse der Durchmesser des Faßkragens 5 eine Fehlermeldung erfolgen, da die Meßeinrichtungen 12 einerseits feststellen werden, daß der Faßkragen 5 nicht horizontal steht und andererseits die Kamera 3 ein ellipsenförmiges Bild des Faßkragens 5 aufnehmen wird, das mit den dann ermittelten Bestkreisen 6, 7 nicht übereinstimmt. Für einen solchen Fall wird vorgeschlagen, daß vier, um jeweils 90° zueinander versetzte Referenzpunkte 14 auf der Bodenfläche 4 des Fasses 1 aufgenommen werden (vgl. Fig. 2 und 3) und der Abstand der Referenzpunkte 14 von der horizontal liegenden Referenzebene 11 bestimmt wird. Da die Bodenfläche 4 des Fasses 1 durch den Faßkragen 5 vor Deformierungen geschützt ist, ist bei einem normalen Faß 1 davon auszugehen, daß die Bodenfläche 4 horizontal liegt. Wird bei der Abstandsmessung der Referenzpunkte 14 auf der Bodenfläche 4 festgestellt, daß die einzelnen Referenzpunkte 14 unterschiedliche Abstände zu der Referenzebene 11 aufweisen, so kann rechnerisch die Neigung der Bodenfläche 4 und damit des Fasses 1 ermittelt werden. Die so ermittelte Neigung des Fasses 1 wird als Korrekturfaktor bei der Abstandsmessung der Meßeinrichtungen 12 sowie der Ermittlung der Bestkreise 6, 7 eingearbeitet. Dadurch kann die Schiefstellung des Fasses 1 kompensiert werden. Erst nach dieser Kompensation wird die Auswertung der Durchmesser in Bezug auf die Bestkreise 6, 7 durchgeführt, so daß Fehlmessungen und ungerechtfertigte Aussonderungen des Fasses 1 vermieden werden.

Das Faß 1 wird über ein Faßventil oder Fitting 15 gereinigt und befüllt. Das Fitting 15 ist bspw. über eine Muffe 16 in der Bodenfläche 4 des Fasses 1 eingeschraubt. Beim Transport des Fasses 1 kann die Muffe 16 deformiert werden, was zu einer Schiefstellung des Fittings 15 führt. In einem solchen Fall ist eine ordnungsgemäße Reinigung, Befüllung oder Entleerung nicht zu gewährleisten, so daß das Faß 1 auszusondern ist. Ein Faß 1 mit einer derart deformierten Muffe 16 und schiefstehendem Fitting 15 ist in Fig. 9 dargestellt. Um die Schiefstellung des Fittings 15 ermitteln zu können, werden wenigstens zwei, einander vorzugsweise gegenüberliegende Referenzpunkte 17 (vgl. Fig. 2 und 3) auf der Oberfläche des Fittings 15 aufgenommen und ihr Abstand zu der Bodenfläche 4 des Fasses 1 ermittelt. Ergibt sich für die beiden Referenzpunkte 17 ein unterschiedlicher Abstandswert zu der möglicherweise geneigten Bodenfläche 4 des Fasses 1, so wird das Faß 1 ausgesondert.

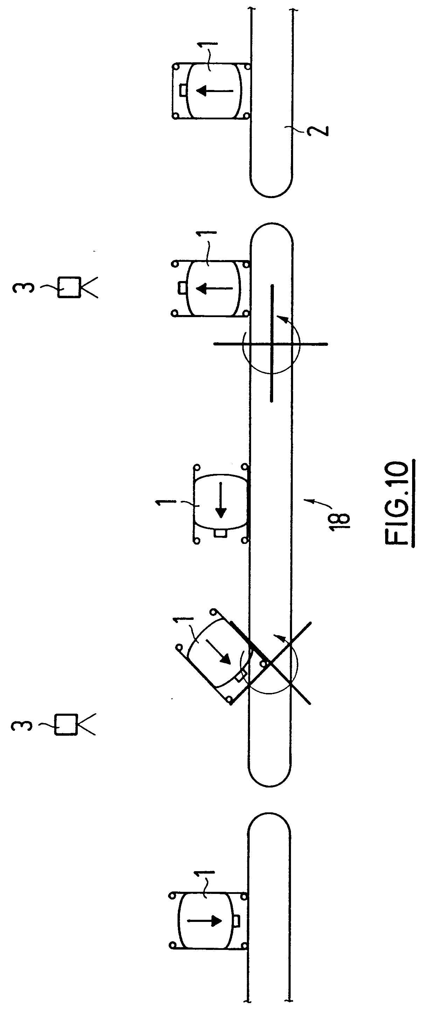

Da eine Überprüfung des Fasses 1 sowohl auf seiner Unterseite als auch seiner Oberseite erfolgen sollte, ist bei einer erfindungsgemäßen, in Fig. 10 schematisch dargestellten Vorrichtung vorgesehen, daß eine Kamera 3 sowohl vor als auch hinter einer Wendemaschine 18 der Abfüllanlage angeordnet ist. Eine solche Wendemaschine 18 ist in den Abfüllanlagen ohnehin vorhanden, da die Fässer 1 üblicherweise mit dem Fitting 15 nach oben auf Paletten in die Abfüllbetriebe zurückgebracht werden. Dort werden sie entpalettiert und mit Fitting 15 nach oben auf dem Transportband 2 in eine Behandlungsanlage gebracht. Da der Reinigungs- und/oder Füllvorgang üblicherweise bei nach unten gekehrtem Fitting 15 durchgeführt wird, werden die Fässer 1 in der Wendemaschine 18 gewendet und mit Fitting 15 nach unten auf das Transportband 2 gesetzt. Durch Anordnung je einer Kamera 3 vor und nach der Wendemaschine 18 kann somit ohne zusätzlichen Aufwand sowohl die Unter- als auch die Oberseite des Fasses 1 untersucht werden. Die Datenverarbeitungseinrichtung kann einen Zwischenspeicher für die Daten des von der vor der Wendemaschine 18 angeordneten Kamera 3 aufgenommenen Bildes aufweisen, so daß die Daten der Ober- und Unterseite des Fasses 1 gemeinsam ausgewertet werden können. Wird bei der Ober- oder Unterseite des Fasses 1 eine Deformation des Faßkragens 5 und/oder des Fittings 15 festgestellt, so wird dies von der Datenverarbeitungseinrichtung an die Anlagensteuerung gemeldet und das Faß 1 über einen hinter der Wendemaschine 18 vorgesehenen in der Zeichnung nicht dargestellten Ausstoßer aus dem Produktionsfluß der Anlage ausgesondert und einem Reparaturbereich zugeführt. Da gleich zu Beginn der Abfüllanlage sowohl die Ober- als auch die Unterseite des Fasses 1 untersucht wird, ist lediglich ein Ausstoßer erforderlich. Gleichzeitig ist sichergestellt, daß vor Beginn des Reinigungs- oder Füllprozesses alle deformierten Fässer ausgesondert werden, so daß hierdurch keine Störungen des Produktionsflusses hervorgerufen werden.

Neben den oben beschriebenen Anwendung zur Analyse von Faßdeformationen kann die Kamera 3 auch zur Überprüfung weiterer Faßeigenschaften verwendet werden. So läßt sich mit der Kamera 3 feststellen, ob das Faß 1 ein Etikett oder dgl. mit Informationen bspw. über das Mindesthaltbarkeitsdatum der in dem Faß 1 enthaltenen Getränkeflüssigkeit aufweist. Fehlt ein solches Etikett, so wird das Faß 1 ausgesondert. Außerdem lassen sich mit der Kamera 3 ggf. eine Leckage des Fittings 15 oder beliebige andere, optisch erfaßbare Informationen ermitteln.

### Bezugszeichenliste:

- 1: Faß
- 2: Transportband
- 3: Kamera
- 4: Bodenfläche
- 5: Faßkragen
- 6: Außenbestkreis
- 7: Innenbestkreis
- 8: Pixel
- 9: Eindellung
- 10: Lücke
- 11: Referenzebene
- 12: Meßeinrichtungen
- 13: Lichtschranke
- 14: Referenzpunkte
- 15: Fitting
- 16: Muffe
- 17: Referenzpunkte
- 18: Wendemaschine

## Patentansprüche

1. Verfahren zur Überprüfung von Gebinden, insbesondere von Fässern wie Kegs, bei dem mit wenigstens einer Kamera (3) ein Bild eines zu überprüfenden Abschnitts des Fasses (1), insbesondere einer Bodenfläche (4) mit einem Faßkragen (5), aufgenommen wird, bei dem die Bilddaten erfaßt und an eine Datenverarbeitungseinrichtung weitergeleitet und in der Datenverarbeitungseinrichtung ausgewertet werden, **dadurch gekennzeichnet,** daß an mehreren, vorzugsweise gleichmäßig über den Umfang des Fasses (1) verteilten Stellen des von der Kamera (3) aufgenommenen Bildes der Außen- und/oder Innendurchmesser des Faßkragens (5) ermittelt und der Faßanalyse zugrundegelegt wird.

2. Verfahren nach Anspruch 1, **dadurch gekennzeichnet,** daß der Außen- und/oder Innendurchmesser des Faßkragens (5) an jeweils 8 Stellen ermittelt wird.

3. Verfahren nach Anspruch 1 oder 2, **dadurch gekennzeichnet,** daß aus den ermittelten Daten des Außen- bzw. Innendurchmessers des Faßkragens (5) ein Mittelwert, der sogenannte Außenbestkreis (6) bzw. Innenbestkreis (7), gebildet wird.

4. Verfahren nach Anspruch 3, **dadurch gekennzeichnet,** daß die Abweichungen des ermittelten maximalen und minimalen Außendurchmessers von dem Außenbestkreis (6) des Faßkragens (5) festgestellt werden.

5. Verfahren nach Anspruch 3 oder 4, **dadurch gekennzeichnet,** daß die Abweichungen des ermittelten maximalen und minimalen Innendurchmessers von dem Innenbestkreis (7) des Faßkragens (5) festgestellt werden.

6. Verfahren nach einem der Ansprüche 3 bis 5, **dadurch gekennzeichnet,** daß der ermittelte Außenbestkreis (6) bzw. Innenbestkreis (7) des Faßkragens (5) mit in einer Datenbank gespeicherten Werten üblicher Fässer verglichen werden.

7. Verfahren nach einem der Ansprüche 1 bis 3, **dadurch gekennzeichnet,** daß in dem von der Kamera (3) aufgenommenen Bild die Anzahl der Pixel (8) ermittelt wird, die außerhalb des Außenbestkreises (6) und innerhalb des Innenbestkreises (7) liegen, und daß die Anzahl der zwischen dem Außenbestkreis (6) und dem Innenbestkreis (7) liegenden Pixel (8) ermittelt wird.

8. Verfahren nach Anspruch 7, **dadurch gekennzeichnet,** daß das Faß (1) ausgesondert wird, wenn in einem Umfangsbereich des Faßkragens (5) keine Pixel (8) ermittelt werden.

9. Verfahren nach einem der Ansprüche 1 bis 8, **dadurch gekennzeichnet,** daß der Abstand verschiedener Umfangsbereiche des Faßkragens (5) zu einer Referenzebene (11) ermittelt wird, die vorzugsweise horizontal liegt.

10. Verfahren nach Anspruch 9, **dadurch gekennzeichnet,** daß die Abstandsmessung mittels eines optischen Lasermeßgerätes oder Ultraschall erfolgt.

11. Verfahren nach Anspruch 9, **dadurch gekennzeichnet,** daß die Abstandsmessung mit 2 Kameras durchgeführt wird und der Abstand durch die Auswertung der Lichtreflexe, bspw. durch Stereometrie, ermittelt wird.

12. Verfahren nach einem der Ansprüche 1 bis 11, **dadurch gekennzeichnet,** daß auf der Bodenfläche (4) des Fasses (1) Referenzpunkte (14) aufgenommen werden, und daß der Abstand der Referenzpunkte (14) von der Referenzebene (11) ermittelt wird.

13. Verfahren nach Anspruch 12, **dadurch gekennzeichnet,** daß 4, vorzugsweise um jeweils 90° zueinander versetzte Referenzpunkte (14) auf der Faßbodenfläche (4) aufgenommen werden.

14. Verfahren nach Anspruch 12 oder 13, **dadurch gekennzeichnet,** daß der Abstand der einzelnen Referenzpunkte (14) auf der Faßbodenfläche (4) von der Referenzebene (11) zueinander in Beziehung gesetzt wird, daß die Relativlage der Faßbodenfläche (4) zur Horizontalen ermittelt wird, und daß bei einer ermittelten Schiefstellung des Fasses (1) das von der Kamera (3) aufgenommene Bild des Faßkragens (5) um die Faßneigung korrigiert wird.

15. Verfahren nach einem der Ansprüche 1 bis 14, **dadurch gekennzeichnet,** daß von der Ober- und/oder der Unterseite des Fasses (1) ein Bild aufgenommen und ausgewertet wird, und daß das Faß (1) ausgesondert wird, wenn die ermittelten Daten der Ober- und/oder Unterseite des Fasses (1) außerhalb bestimmter Toleranzbereiche liegen.

16. Verfahren nach einem der Ansprüche 1 bis 15, **dadurch gekennzeichnet,** daß der Abstand der Oberfläche eines Fittings (15) von der Faßbodenfläche (4) an wenigstens zwei, einander vorzugsweise gegenüberliegenden Punkten der Fittingoberfläche ermittelt wird, und daß das Faß (1) ausgesondert wird, wenn die Abstände der wenigstens zwei Punkte der Fittingoberfläche von der Faßbodenfläche (4) um mehr als einen festgelegten Toleranzwert voneinander abweichen.

17. Vorrichtung zur Durchführung eines Verfahrens nach einem der Ansprüche 1 bis 16, mit wenigstens einer Kamera (3) zur Aufnahme von Bildern von zu überprüfenden Abschnitten der Fässer (1), insbesondere der Bodenfläche (4) des Fasses (1) mit dem Faßkragen (5), und einer Datenverarbeitungseinrichtung, in der die Bilddaten ausgewertet werden **gekennzeichnet durch** eine Meßeinrichtung (12) für die Ermittlung des Abstandes einzelner Abschnitte des Fasses (1) von einer Referenzebene (11).

18. Vorrichtung nach Anspruch 17, **dadurch gekennzeichnet,** daß die Kamera (3) eine CCD-Zeilenkamera ist.

19. Vorrichtung nach Anspruch 17, **dadurch gekennzeichnet,** daß die Kamera (3) eine Videokamera ist.

20. Verfahren nach einem der Ansprüche 17 bis 19, **dadurch gekennzeichnet,** daß die Datenverarbeitungseinrichtung einen Speicher zur Speicherung der ermittelten Daten und/oder einen Speicher für die Daten bekannter Faßtypen aufweist.

21. Vorrichtung nach Anspruch 17, **dadurch gekennzeichnet,** daß die Meßeinrichtung (12) ein optisches Lasermeßgerät oder ein Ultraschallgerät ist.

22. Vorrichtung nach einem der Ansprüche 17 bis 21, **dadurch gekennzeichnet,** daß vor und nach einer Wendemaschine (18) für das Faß (1) jeweils eine Kamera (3) angeordnet ist, die Bilder von der Ober- bzw. der Unterseite des Fasses (1) aufnimmt.

23. Vorrichtung nach Anspruch 22, **gekennzeichnet durch** einen Zwischenspeicher für die Daten des von der vor der Wendemaschine (18) angeordneten Kamera (3) aufgenommenen Bildes.
